# EUROPEAN PATENT SPECIFICATION

(11) **EP 2 087 292 B1**
(45) Date of publication and mention of the grant of the patent: **21.08.2013**
(21) Application number: 07827998.1
(22) Date of filing: 07.11.2007
(51) Int. Cl.: F24F 1/00, F24F 3/12, A61L 9/14

(54) **AIR CONDITIONER**
KLIMAANLAGE
CLIMATISEUR

(30) Priority: 27.11.2006 JP 2006319068
(43) Date of publication of application: 12.08.2009
(73) Proprietor: Panasonic Corporation, Osaka 571-8501 (JP)
(72) Inventor: MIHARA, Fumio, Osaka 540-6207 (JP); SUGAWA, Akihide, Osaka 540-6207 (JP)
(74) Representative: Appelt, Christian W.
(86) International application number: PCT/JP2007/001220
(87) International publication number: WO 2008/065737

(56) References cited:
- EP-A- 1 619 447
- JP-A- 2004 358 358
- JP-A- 2006 061 408
- JP-A- 2006 088 123
- JP-A- 2006 151 046
- JP-A- 2006 234 245

## Description

### Technical Field

The present invention is directed to an air conditioner which is configured to blow an air flow carrying a mist of charged minute water particles for deodorizing a room environment.

### Background Art

Japanese Patent Publication No. 2006-234245 discloses an air conditioner which incorporates an atomizing unit which generates a mist of the charged minute water particles for carrying the mist on an air flow blown from the air conditioner. In the air conditioner, the atomizing unit is disposed in the course of an air flow to give considerable flow resistance, thereby lowering air flow efficiency.

From EP 1 619 447 A2, an air conditioning unit 1 having a housing 3 is known. The unit comprises a turbulence generator 11 having an air moisturizer 26.

### DISCLOSURE OF THE INVENTION

In view of the above problem, the present invention is accomplished to provide an improved air conditioner which is capable of exhibiting the deodorizing effect, yet without lowering the air flow efficiency. The air conditioner in accordance with the present invention includes a housing incorporating a blower and an atomizing unit. The housing is provided with an air inlet, an air outlet, and an internal flow channel leading from the air inlet to the air outlet. The blower is disposed in the flow channel and is configured to draw the air through the air inlet and blow the air out through the air outlet. The atomizing unit is configured to be supplied with water and to electrostatically atomize it into a mist of minute charged water particles. The mist is discharge out of a feed port of the atomizing unit. The housing has a partition which divides an interior of the housing into the flow channel and a compartment within which the atomizing unit is mounted. A feed conduit extends from the feed port of the atomizing unit into the flow channel through the partition to feed the mist to the air being blown out through the air outlet. Thus, the atomizing unit is isolated from the flow channel to minimize flow resistance, thereby assuring efficient air flow carrying the mist thereon for deodorization over a wide space in the room.

Preferably, the feed conduit is made of a flexible material to be bent intermediate at its opposite ends so that it can be routed from the atomizing unit to an optimum point in the flow channel for improving design flexibility

Further, the air outlet of the housing is preferred to be provided with a louver to which the feed conduit extends for merging the mist of the charged minute water particles in the air just leaving the air outlet and directed to a particular direction. Thus, the mist can be conveyed along a desired direction for improving the deodorizing effect.

In this connection, one of slats of the louver is preferred to be made movable about a pivot axis and to include a duct which a connector for the feed conduit. The connector is aligned with the pivot axis and is configured to catch the feed conduit in such a manner that the feed conduit is rotatable relative to the connector about the pivot axis. Thus, the mist can be directed in a desired direction defined by the angle of the movable slat.

### Brief Description of the Drawings

[fig.1]FIG. 1 is a side section of an air conditioner in accordance with a preferred embodiment of the present invention;
[fig.2]FIG. 2 is a perspective view of an atomizing unit employed in the above air conditioner;
[fig.3]FIG. 3 is a sectional view of the above atomizing unit;
[fig.4]FIG. 4 is a sectional view of a principal part of the above atomizing unit;
[fig.5]FIG. 5 is a perspective view of a slat utilized in the above conditioner; and
[fig.6]FIG. 6 is a perspective view of the slat shown at an angle different from that of FIG. 5.

### Best Mode for Carrying Out the Invention

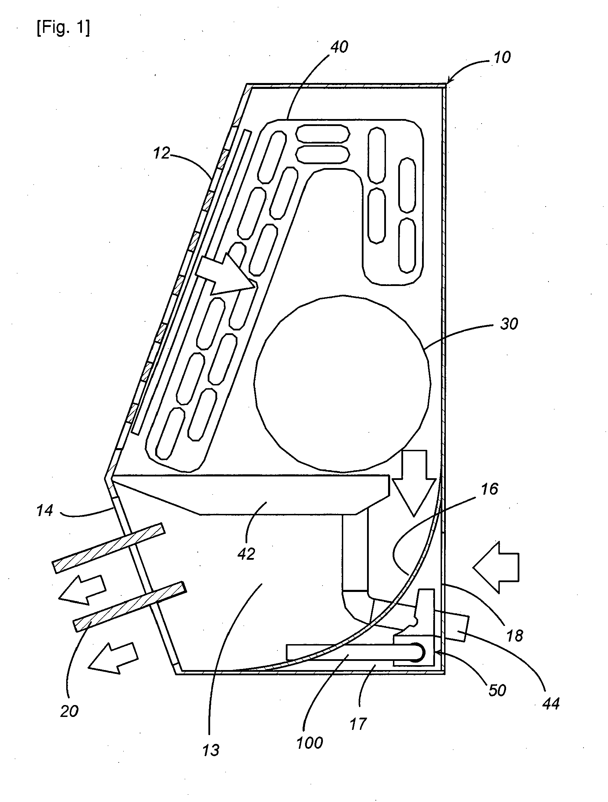

Referring now to FIG. 1, there is shown an air conditioner in accordance with a preferred embodiment of the present invention. The air conditioner includes a housing 10 incorporating a blower 30, a heat exchanger 40, and an atomizing unit 50. The housing 10 is formed in its front wall with an air inlet 12 and an air outlet 14 respectively in its upper and lower parts of the front wall. The blower 30 is disposed in a flow channel leading from the air inlet 12 to the air outlet 14 to draw the air through the air inlet and blow it out through the air outlet. The heat exchanger 40 is disposed in the flow channel upstream of the blower to cool or heat the air being blown out into a room.

A partition 16 is formed in the housing 10 to divide an interior space of the housing into a flow channel 13 leading from the air inlet 12 to the air outlet 14, and a compartment 17 accommodating therein the atomizing unit 50. The atomizing unit 50 is configured to generate a mist of charged minute water particles which is carried on the air being blown out of the air outlet 14. A drip pan 42 is disposed below the heat exchanger 40 and the blower 30 to receive droplets of condensed water dripping from the heat exchanger 40. The water is drained out through a drain pipe 44 extending through a rear wall of the housing 10. The drip pan 42 has its rear end terminated in a spaced relation with the rear wall to define therebetween a gap through which the air is directed from the blower 30 towards the partition 16. The partition 16 is arcuately curved to direct the air to the air outlet 14 for smoothly blowing the air out through the air outlet 14.

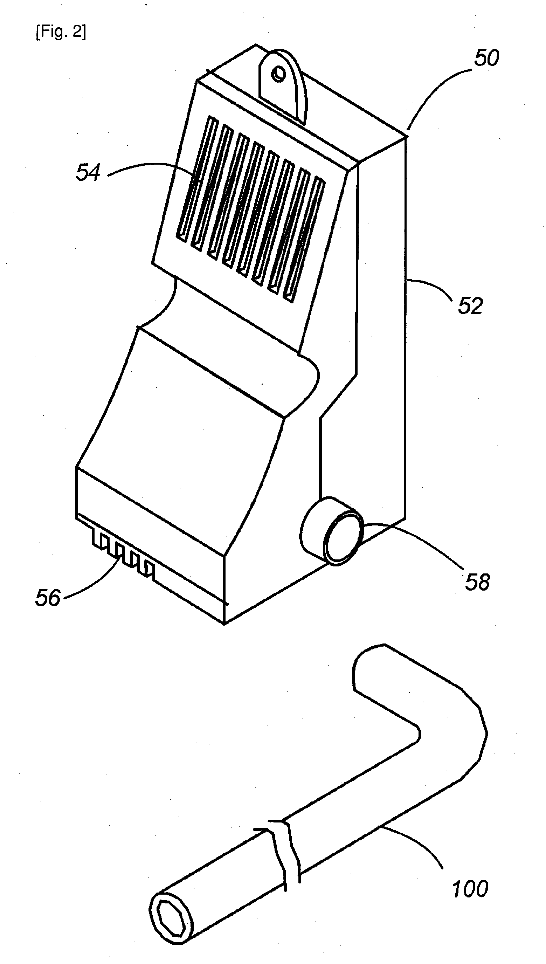

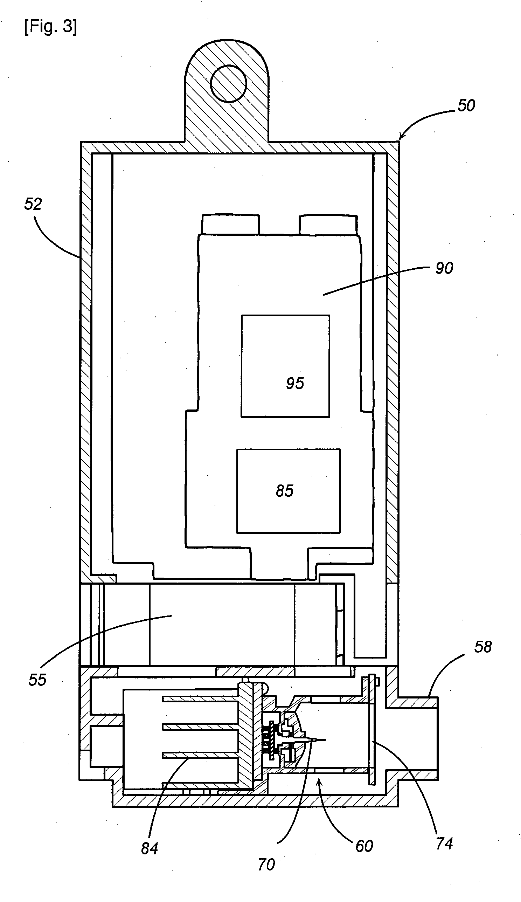

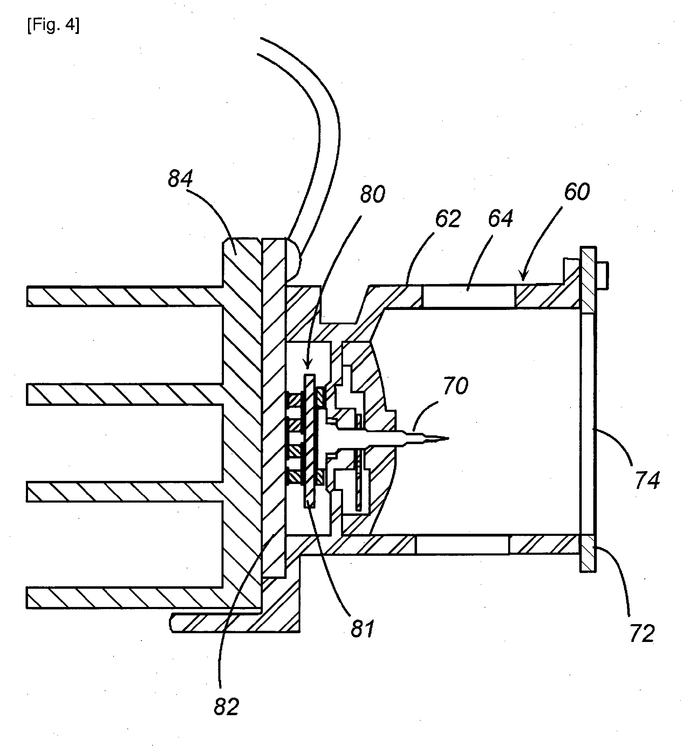

As shown in FIGS. 2 to 4, the atomizing unit 50 includes an atomizer 60 disposed in a casing 52 with a top air intake 54 and a bottom air exit 56. The atomizer 60 is disposed in a bottom of the casing 52 and includes a cylindrical barrel 62 carrying an emitter electrode 70 and an opposed electrode 72. The emitter electrode 70 projects in the center of the barrel 62, while the opposed electrode 72 is fixed at the front end of the barrel 62 in an opposite relation to the emitter electrode 70. The opposed electrode 72 is shaped from an electrically conductive substrate with a circular opening 74 which has an inner periphery spaced from a tip of the emitter electrode 70 to define a discharge port of discharging the mist. The atomizer 60 includes cooling means 80 and a high voltage source 95. The cooling means 80 is coupled to cool the emitter electrode 70 in order to condense the water content carried in the surrounding air on the emitter electrode 70, thereby supplying the water thereto. The high voltage source 95 is configured to apply a high voltage across the emitter electrode 70 and the opposed electrode 72 so as to charge the water on the emitter electrode 70 and atomize it into the charged minute water particles to be discharged out through the circular opening 74, i.e., the discharge port of the atomizer.

The cooling means 80 is realized by a Peltier module having a cooling side coupled to the emitter electrode 70, and having thermo-electric elements which, upon being applied with a predetermined voltage, cools the emitter electrode to a temperature below a dew point of the water. The Peltier module has a plurality of thermo-electric elements arranged in parallel with each between thermal conductors 81 and 82 to cool the emitter electrode 70 at a cooling rate determined by a variable voltage given from a cooling electric source circuit 85. One thermal conductor 81 defining the cooling side is coupled to the emitter electrode 70, while the other thermal conductor 82 defining the heat radiation side is provided with a heat radiator 84. The Peltier module is fixed between the bottom of the barrel 62 and the heat radiator 84 with its cooling side conductor 81 in heat transfer contact with a root of the emitter electrode 70. The high voltage source 95 is configured to apply a predetermined high voltage across the emitter electrode 70 and the grounded opposed electrode to give a negative or positive voltage (for example, - 4.6 kV) to the emitter electrode 70. The cooling electric source 85 and the high voltage source 95 are packed in a circuit module 90 disposed in an upper portion of the casing 52. The atomizing unit 50 further includes a cooling fan 55 which is configured to draw the air from the air intake 54 and expel it out of the air exit 56 for cooling the circuit module 90 and the heat radiator 84. The resulting air flow is partly introduced into the inside of the barrel 62 of the atomizer 60 through openings 64 of the barrel 62 for condensing the water content of the air on the emitter electrode 70. In this connection, the housing 10 is formed in its rear wall with an opening 18 through which the air is taken into the casing 52 of the atomizing unit 50. The air flow generated by the cooling fan 55 is also utilized to discharge the mist out of the opening 74 of the opposed electrode 72.

The casing 52 is provided at its lower end with a feed port 58 which is aligned with the opening 74 of the atomizer 60 and is coupled with a feed conduit 100 for feeding the mist of the charged minute water particles to the air being blown out through the air outlet 14. The feed conduit 100 is bent intermediate at its opposite ends to project its one end through the partition 16 into the flow channel 13 of the housing 10. The feed conduit 100 is made of a flexible material so as to be bent at a desired angle and kept the angled shape for directing the mist along a desired direction towards the air outlet 14 of the housing 10. The flexible material may be selected from antistatic plastic materials including ABS, PS, PP, and PA.

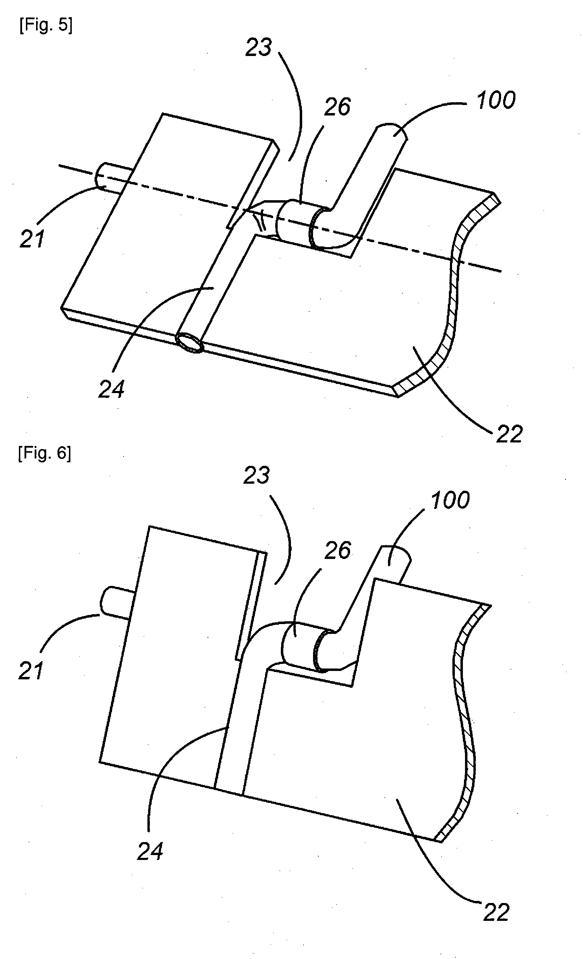

The air outlet 14 is provided with a louver 20 having a plurality of movable slats 22 each configured to pivot about a horizontal pivot axis for deflecting the air blown out through the air outlet 14. As shown in FIGS. 5 and 6, one of the slats 21 may be provided with a duct 24 directing the mist to the air just leaving the air outlet 14. The duct 24 is embedded in the slat 22 and has its front opening exposed at a front edge of the slat 22, and has is rear end projected in a recess 23 at the rear edge of the slat 22. The rear end of the duct 24 is fixed to a tubular connector 26 which is aligned with the pivot axis defined by pivot pins 21 projecting on opposite sides of the slat 22. The connector 26 is configured to receive the front end of the conduit 100 in such a manner that the connector 26 can move together with the slat 22 relative to the conduit 100 about the pivot axis. Thus, the duct 100 moves together with the slat 22 to direct the mist in the direction determined by the angle of the slat 22. While the slat 22 rotates about the pivot axis, the recess 23 is responsible for avoiding the contact of the slat 22 with the connector 26 and the associated portion of the conduit 100.

In a modification of the above embodiment, the conduit 100 may be configured to extend to the air outlet 14 at a position not interfering with the slats of the louver 20.

## Claims

1. An air conditioner comprising:
a housing configured to have an air inlet (12), an air outlet (14), and an internal flow channel (13) leading from said air inlet to said air outlet;
a blower (30) disposed in said flow channel within said housing and configured to draw the air through said air inlet and blow the air out through said air outlet;
an atomizing unit (50) configured to be supplied with water and to electrostatically atomize it into a mist of minute charged water particles, said atomizing unit having a feed port (58) for discharging said mist;
being **characterised by**
said housing having a partition (16) which divides an interior of said housing into said flow channel and a compartment (17) within which said atomizing unit is mounted,
wherein a feed conduit (100) extends from said feed port of the atomizing unit into said flow channel through said partition to feed the mist to the air being blown out through said air outlet,
said feed conduit (100) being made of antistatic plastic materials and being bent at a desired angle and kept the angled shape for directing the mist along a desired direction towards said air outlet (14) of the housing (10).

2. An air conditioner as set forth in claim 1, wherein
said feed conduit is configured to be capable of being bent at a portion intermediate its opposite ends.

3. An air conditioner as set forth in claim 1, wherein
said feed conduit extends into a louver (20) provided at said air outlet.

4. An air conditioner as set forth in claim 1, wherein
said air outlet is provided with a louver (20) including a slat (22) movable about a pivot axis, said slat having a duct with a connector (26) for said feed conduit, said connector being aligned with said pivot axis and configured to catch said feed conduit in such a manner that said feed conduit is rotatable relative to said connector about said pivot axis.

5. An air conditioner as set forth in claim 4, wherein
said duct is embedded in said slat to dispose its front opening at a front edge of said slant and to have its rear opening coupled to feed conduit by means of said connector.

## Patentansprüche

1. Eine Klimaanlage, die aufweist:
ein Gehäuse, das so ausgebildet ist, dass es einen Lufteinlass (12), einen Luftauslass (14) und einen von dem Lufteinlass zu dem Luftauslass führenden inneren Strömungskanal (13) umfasst,
ein in dem Strömungskanal in dem Gehäuse angeordnetes Gebläse (30), das eingerichtet ist, die Luft durch den Lufteinlass anzusaugen und die Luft durch den Luftauslass auszublasen;
eine Zerstäubereinheit (50), die eingerichtet ist, Wasser zugeführt zu bekommen und dieses elektrostatisch in einen Dunst winziger geladener Wasserpartikel zu zerstäuben, wobei die Zerstäubereinheit eine Einspeiseöffnung (58) zum Abgeben des Dunstes aufweist,
**dadurch gekennzeichnet, dass**
das Gehäuse eine Abtrennung (16) aufweist, die ein Inneres des Gehäuses in den Strömungskanal und eine Kammer (17) teilt, in der die Zerstäubereinheit montiert ist,
wobei eine Zufuhrleitung (100) sich von der Einspeiseöffnung der Zerstäubereinheit durch die Abtrennung in den Strömungskanal erstreckt, um den Dunst der Luft zuzuführen, die durch die Auslassöffnung ausgeblasen wird,
wobei die Zufuhrleitung (100) aus antistatischen Kunststoffmaterialien gefertigt und in einem gewünschten Winkel gekrümmt ist, und die abgewinkelte Form beibehalten wird, um den Dunst in eine gewünschten Richtung zum Luftauslass (14) des Gehäuses zu richten.

2. Eine Klimaanlage gemäß Anspruch 1, wobei
die Zufuhrleitung eingerichtet ist, in einem Bereich zwischen ihren entgegengesetzten Enden gekrümmt werden zu können.

3. Eine Klimaanlage gemäß Anspruch 1, wobei
die Zufuhrleitung sich in ein Gitter (20) erstreckt, das an der Auslassöffnung vorgesehen ist.

4. Eine Klimaanlage gemäß Anspruch 1, wobei die Auslassöffnung über ein Gitter (20) verfügt, das einen um eine Drehachse beweglichen Flügel (22) aufweist, wobei der Flügel einen Durchlass mit einem Verbindungselement (26) für die Zufuhrleitung aufweist, wobei das Verbindungselement gemäß der Drehachse ausgerichtet und konfiguriert ist, die Zufuhrleitung derart einzufassen, dass die Zufuhrleitung in Bezug auf das Verbindungselement um die Drehachse drehbar ist.

5. Eine Klimaanlage gemäß Anspruch 4, wobei
der Durchlass so in den Flügel eingelassen ist, dass seine vordere Öffnung an einer-Vorderkante des Flügels angeordnet und seine hintere Öffnung mittels des Verbindungselements an die Zufuhröffnung angeschlossen werden kann.

## Revendications

1. Climatiseur comprenant :
un logement configuré de sorte à avoir une entrée d'air (12), une sortie d'air (14) et un canal d'écoulement interne (13) menant de ladite entrée d'aïr à ladite sortie d'air;
un ventilateur soufflant (30) disposé dans ledit canal d'écoulement à l'intérieur dudit logement et configuré pour attirer l'air à travers ladite entrée d'air et évacuer l'air à travers ladite sortie d'air ;
une unité d'atomisation (50) configurée pour être alimentée en eau et pour l'atomiser électrostatiquement sous forme de brume de petites particules d'eau chargées, ladite unité d'atomisation comprenant un orifice d'alimentation (58) pour diffuser ladite brume ;
étant **caractérisé en ce que**
ledit logement comportant une cloison (16) qui divise un intérieur dudit logement en ledit canal d'écoulement et un compartiment (17) à l'intérieur duquel est montée ladite unité d'atomisation,
dans lequel un conduit d'alimentation (100) s'étend à partir dudit orifice d'alimentation de l'unité d'atomisation dans ledit canal d'écoulement à travers ladite cloison pour fournir la brume à l'air soufflé à travers ladite sortie d'air,
ledit conduit d'alimentation (100) étant fabriqué à partir de matières plastiques antistatiques et étant courbé selon un angle souhaité et maintenu incliné pour diriger la brume le long d'une direction souhaitée vers ladite sortie d'air (14) du logement (10).

2. Climatiseur selon la revendication 1, dans lequel
ledit conduit d'alimentation est configuré pour pouvoir être courbé au niveau d'une partie située entre ses extrémités opposées.

3. Climatiseur selon la revendication 1, dans lequel
ledit conduit d'alimentation s'étend dans un évent (20) prévu au niveau de ladite sortie d'air.

4. Climatiseur selon la revendication 1, dans lequel
ladite sortie d'air est dotée d'un évent (20) comprenant une lame (22) mobile autour d'un axe d'articulation, ladite lame comprenant un tuyau avec un raccord (26) pour ledit conduit d'alimentation, ledit raccord étant aligné sur ledit axe d'articulation et configuré pour se mettre en prise avec ledit conduit d'alimentation de telle sorte que ledit conduit d'alimentation puisse tourner par rapport audit raccord autour dudit axe d'articulation.

5. Climatiseur selon la revendication 4, dans lequel
Ledit tuyau est logé dans ladite lame de façon à ce que son ouverture avant se trouve au niveau du bord avant de ladite lame et que son ouverture arrière soit couplée au conduit d'alimentation au moyen dudit raccord.
